Europäisches Patentamt

European Patent Office

Office européen des brevets

(19)

(11) Veröffentlichungsnummer: **0 415 484 A2**

## (12) EUROPÄISCHE PATENTANMELDUNG

(21) Anmeldenummer: 90202255.7

(22) Anmeldetag: 22.08.90

(51) Int. Cl.5: **G03B 42/02**, G21K 1/02

(30) Priorität: 26.08.89 DE 3928282

(43) Veröffentlichungstag der Anmeldung:
06.03.91 Patentblatt 91/10

(84) Benannte Vertragsstaaten:
**DE FR GB**

(71) Anmelder: **Philips Patentverwaltung GmbH
Wendenstrasse 35 Postfach 10 51 49
W-2000 Hamburg 1(DE)**

(84) **DE**

Anmelder: **N.V. Philips' Gloeilampenfabrieken
Groenewoudseweg 1**

**NL-5621 BA Eindhoven(NL)**

(84) **FR**

(72) Erfinder: **Conrads, Norbert
Stock 9
B-4729 Hauset(BE)**
Erfinder: **Hillen, Walter, Dr.
Dinkermichsweg 38
W-5100 Aachen-Walheim(DE)**

(74) Vertreter: **von Laue, Hanns-Ulrich et al
Philips Patentverwaltung GmbH,
Wendenstrasse 35, Postfach 10 51 49
W-2000 Hamburg 1(DE)**

(54) **Röntgenaufnahmevorrichtung.**

(57) Bei einer Röntgenaufnahmevorrichtung mit einer Röntgenstrahlenquelle (1), deren Strahlung durch eine schlitzförmige Öffnung wenigstens einer Primärblende fächerförmig auf ein Untersuchungsobjekt (5) trifft und zeilenweise über dieses geführt wird, ist zur Feldeinblendung vorgesehen, daß die Primärblende wenigstens zwei Blendenelemente (4, 4) aufweist, die in Normalposition die schlitzförmige Öffnung bilden und die zur Festlegung eines Untersuchungsbereichs quer zur Schlitzrichtung auf je eine wählbare Begrenzungsposition einstellbar sind. Es ist ferner eine Lichtquelle (3) vorgesehen, deren Lichteinfall auf das Untersuchungsobjekt (5) mittels der in den Begrenzungspositionen befindlichen Blendenelemente (4, 4) begrenzt wird und so den Untersuchungsbereich markiert. Eine Belichtung des Untersuchungsobjektes (5) mit Röntgenstrahlen wird nur zwischen den beiden Positionen der Primärblende vorgenommen, in denen sich deren Blendenelemente in ihrer jeweiligen Begrenzungsposition finden.

FIG.1

EP 0 415 484 A2

# RÖNTGENAUFNAHMEVORRICHTUNG

Die Erfindung betrifft eine Röntgenaufnahmevorrichtung mit einer Röntgenstrahlquelle, deren Strahlung durch eine schlitzförmige Öffnung wenigstens einer Primärblende fächerförmig auf ein Untersuchungsobjekt trifft und zeilenweise über dieses geführt wird, und mit einem Strahlungsdetektor.

In der Radiografie besteht im allgemeinen der Wunsch, den Untersuchungsbereich, d.h. also denjenigen Bereich, in dem ein Untersuchungsobjekt mit Röntgenstrahlen belichtet wird, vor der Belichtung kenntlich zu machen. Diese Festlegung des Untersuchungsbereichs geschieht im allgemeinen dadurch, daß zwischen Fokus der Röntgenstrahlenquelle und einer feststehenden Blende Lichtstrahlen eingeblendet werden, welche durch die Blende begrenzt werden und so in der Untersuchungsebene den Untersuchungsbereich kennzeichnen.

In der spaltradiografischen Belichtungstechnik ist dies jedoch nicht ohne weiteres möglich, da hier die Primärblende nur einen schmalen Spalt aufweist, welcher dazu dient, die Röntgenstrahlung fächerförmig zeilenweise bzw. bereichsweise über das Untersuchungsobjekt zu führen.

Eine andere Möglichkeit der Lichteinblendung besteht bei der spaltradiografischen Belichtungstechnik darin, daß Licht zwischen den Primärblende und dem Untersuchungsobjekt einzuspiegeln. Eine solche Anordnung hat jedoch den Nachteil, daß damit der Abstand zwischen dem Fokus der Röntgenstrahlenquelle und der Primärblende zwangsweise verringert wird, wenn die Gesamtanordnung nicht zu groß werden soll. Der Abstand zwischen Fokus und Primärblende sollte jedoch möglichst groß sein, um die Halbschattenbereiche des Strahlenfächers in der Untersuchungsebene möglichst klein zu halten. Dies wiederum ist wünschenswert aus Gründen der Streustrahlenunterdrückung und der Primärstrahlentransmission. Darüber hinaus wird durch die Lichteinblendung zwischen Untersuchungsebene und Primärspalt der Arbeitsbereich zur Objektpositionierung in der Untersuchungsebene eingeschränkt.

Es ist Aufgabe der Erfindung, eine Röntgenaufnahmevorrichtung der eingangs genannten Art dahingehend auszugestalten, daß eine Feldeinblendung unter Vermeidung der oben beschriebenen Nachteile möglich ist.

Diese Aufgabe ist erfindungsgemäß dadurch gelöst, daß die Primärblende wenigstens zwei Blendenelemente aufweist, die in Normalposition die schlitzförmige Öffnung bilden und die zur Festlegung eines Untersuchungsbereichs quer zur Schlitzrichtung auf je eine wählbare Begrenzungsposition einstellbar sind, daß eine Lichtquelle vorgesehen ist, deren Lichteinfall auf das Untersuchungsobjekt mittels der in den Begrenzungspositionen befindlichen Blendenelemente begrenzt wird und so den Untersuchungsbereich markiert und daß eine Belichtung des Untersuchungsobjektes mit Röntgenstrahlen nur zwischen den beiden Positionen der Primärblende vorgenommen wird, in denen sich deren Blendenelemente in ihrer jeweiligen Begrenzungsposition befinden.

Die Primärblende dient bei dieser Anordnung in ihrer Normalposition, in der die beiden Blendenelemente zusammengefahren sind und die schlitzförmige Öffnung bilden, in gewohnter Weise zur spaltradiografischen Belichtung des Untersuchungsobjektes.

Vor dem Untersuchungsvorgang sind jedoch die beiden Blendenelemente quer zur Richtung des durch sie geformten schlitzförmigen Öffnung auseinanderfahrbar. Oberhalb dieser Primärblendenelemente ist eine Lichtquelle vorgesehen, deren Lichteinfall auf das Untersuchungsobjekt mittels der beiden Blendenelemente begrenzt wird. Die Blendenelemente werden nun so weit auseinanderbewegt, daß mittels des Lichteinfalls der gewünschte Untersuchungsbereich markiert wird. Die Blendenelemente befinden sich dann in je einer Begrenzungsposition.

Nach dieser Festlegung des Untersuchungsbereiches werden die Blendenelemente wieder in ihrer Normalposition bewegt, so daß sie wieder die schlitzförmige Öffnung bilden. Bei der Belichtung des Untersuchungsobjektes mittels Röntgenstrahlen wird die Primärblende bewegt, so daß das Untersuchungsobjekt zeilenweise mit den Röntgenstrahlen belichtet wird. Eine solche Belichtung des Untersuchungsobjektes wird jedoch nur zwischen den beiden Positionen der Primärblende vorgenommen, in denen sich deren Blendenelemente ihrer jeweiligen Begrenzungsposition befinden. Die Belichtung mit Röntgenstrahlen wird also begonnen, wenn das erste Blendenelement seine Begrenzungsposition erreicht hat. Dann wird die Primärblende weiterbewegt, bis das zweite Blendenelement seine Begrenzungsposition erreicht hat. Ist dies der Fall, wird die Belichtung mit Röntgenstrahlen abgebrochen.

Bei dieser Anordnung ergibt sich keine Beschränkung des Untersuchungsbereiches, da zwischen Primärblende und Untersuchungsbereich der Arbeitsbereich uneingeschränkt bleibt. Es ist dennoch für eine spaltradiografische Röntgenaufnahmevorrichtung eine Feldeinblendung möglich, bei der in einfacher weise durch Wahl der Begrenzungs-Position der beiden Blendenelemente der Untersuchungsbereich festgelegt und optisch verfolgt werden kann.

Dies kann, wie nach weiteren vorteilhaften Ausgestaltungen der Erfindung vorgesehen ist, entweder dadurch geschehen, daß die beiden Blendenelemente manuell auf die ihnen jeweils zugeordnete Begrenzungsposition einstellbar sind oder daß die beiden Blendenelemente motorisch auf die ihn jeweils zugeordnete Begrenzungsposition einstellbar sind.

Nach einer weiteren Ausgestaltung der Erfindung ist vorgesehen, daß eine Kontrollogik vorhanden ist, welche während einer Aufnahme die Röntgenstrahlenquelle nur während desjenigen Zeitraumes einschaltet, in dem sich die Blendenelemente der Primärblende zwischen den ihnen jeweils zugeordneten Begrenzungspositionen befinden.

Die Kontrollogik sorgt also dafür, daß in Abhängigkeit der gewählten Begrenzungspositionen eine Belichtung des Untersuchungsobjektes nur in dem festgelegten Untersuchungsbereich stattfindet. Je nach Ausführung der Röntgenaufnahmevorrichtung kann es dabei vorgesehen sein, daß mittels entsprechender Bewegung des Primärspaltes in jedem Falle ein maximaler Untersuchungsbereich überstrichen wird. In diesem Falle wird dann jedoch nur während des Zeitraumes, in dem sich die Blendenelemente der Primärblende zwischen den ihnen jeweils zugeordneten Begrenzungsposition finden, die Röntgenstrahlenquelle eingeschaltet.

Ein Ausführungsbeispiel der Erfindung wird anhand der Zeichnung näher erläutert. Es zeigen:
Fig. 1 eine schematische Darstellung der wesentlichen Teile einer Röntgenaufnahmevorrichtung, deren Blendenelemente der Primärblende sich in ihren Begrenzungspositionen befinden,
Fig. 2 die Vorrichtung nach Fig. 1 während des Belichtungsvorganges mit Röntgenstrahlen.

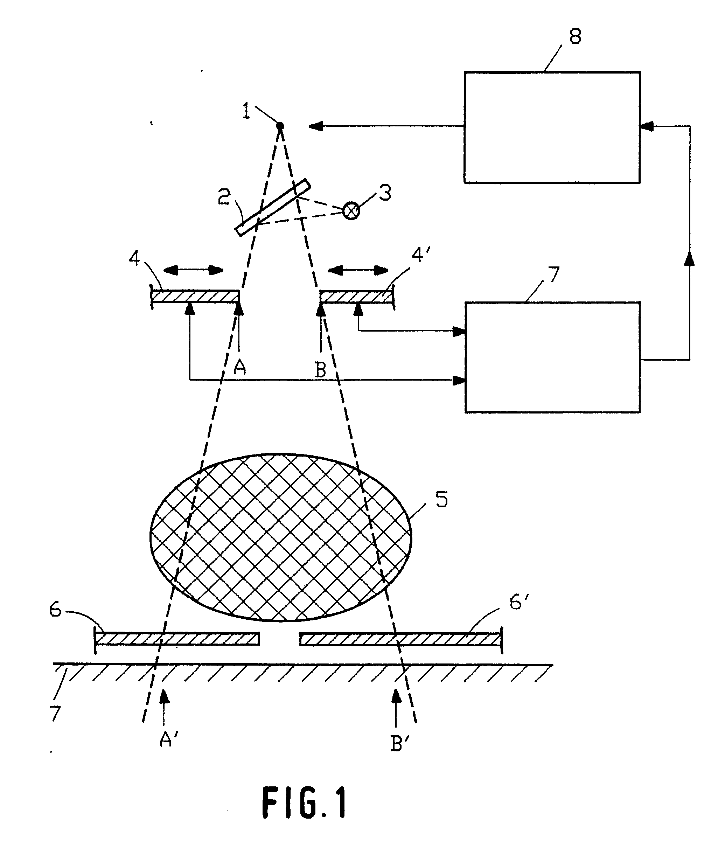

In Fig. 1 ist mit 1 eine Röntgenstrahlenquelle bezeichnet, z.B. eine Röntgenröhre, von der in Fig. 1 jedoch nur der Fokus dargestellt ist, von dem die Röntgenstrahlung ausgeht. Im Strahlengang der Strahlenquelle 1 ist dann ein Spiegel 2 angeordnet, welcher für die von der Strahlenquelle 1 ausgehende Röntgenstrahlung durchlässig ist, mittels dessen jedoch das Licht einer seitlich angeordneten Lichtquelle 3 in den Strahlengang einblendbar ist.

Im Strahlengang ist nachfolgend eine Primärblende angeordnet, welche zwei Blendenelemente 4 und 4′ aufweist, welche sich in der Darstellung in Fig. 1 in jeweils einer Begrenzungsposition befinden, welche in der Figur mit den Pfeilen A und B markiert sind. Die Blendenelemente sind in der Figur nur schematisch und ohne ihre Aufhängung bzw. motorische Steuerung dargestellt.

Im Strahlengang ist nachfolgend in der Figur schematisch ein Untersuchungsobjekt 5 angedeutet, unterhalb dessen eine Sekundärblende vorgesehen ist, welche mittels zwei Blendenelementen 6 und 6′ aufgebaut ist. Die Blendenelemente 6 und 6′ der Sekundärblende bilden eine geradlinige schlitzförmige Öffnung, durch die hindurch Röntgenstrahlen auf einen Röntgenbild-Detektor 7 fallen können, der in der Figur nur schematisch angedeutet ist und bei dem es sich beispielsweise um einen Röntgenfilm oder um einen Fotoleiter handeln kann.

Die beiden Blendenelemente 4 und 4′ der Primärblende sind in in der Figur nicht näher dargestellter Weise motorisch bewegbar, wobei die Steuerung mittels einer Kontrollogik 7 vorgenommen wird, welche einen Röntgengenerator 8 ansteuert, der zum Betreiben der Röntgenstrahlenquelle 1 dient.

Bei der in Fig. 1 gewählten Darstellung befinden sich die beiden Blendenelemente 4 und 4′ zur Festlegung des Untersuchungsbereiches in je einer Begrenzungsposition. Zur Ermittlung des Untersuchungsbereiches ist die Röntgenstrahlenquelle 1 ausgeschaltet, während die Lichtquelle 3 eingeschaltet ist. Das von der Lichtquelle 3 ausgehende Licht wird mittels des Spiegels 2 in den Strahlengang projiziert und gelangt, seitlich begrenzt durch die beiden Blendenelemente 4 und 4′, auf das Untersuchungsobjekt 5 bzw. auf die durch die beiden Sekundär-Blendenelemente 6 und 6′ gebildete Untersuchungsebene. Der Lichteinfall auf das Untersuchungsobjekt 5 bzw. die Untersuchungsebene markiert den Untersuchungsbereich. Auf der Ebene des Untersuchungsbereiches ergeben sich dabei seitliche Begrenzungen A′ und B′, die den Begrenzungspositionen A und B der Primärspalt-Blendenelemente 4 und 4′ entsprechen.

Die beiden Primärspalt-Blendenelemente 4 und 4 können manuell oder motorisch in die in der Fig. 1 dargestellten Begrenzungsposition bewegbar sein.

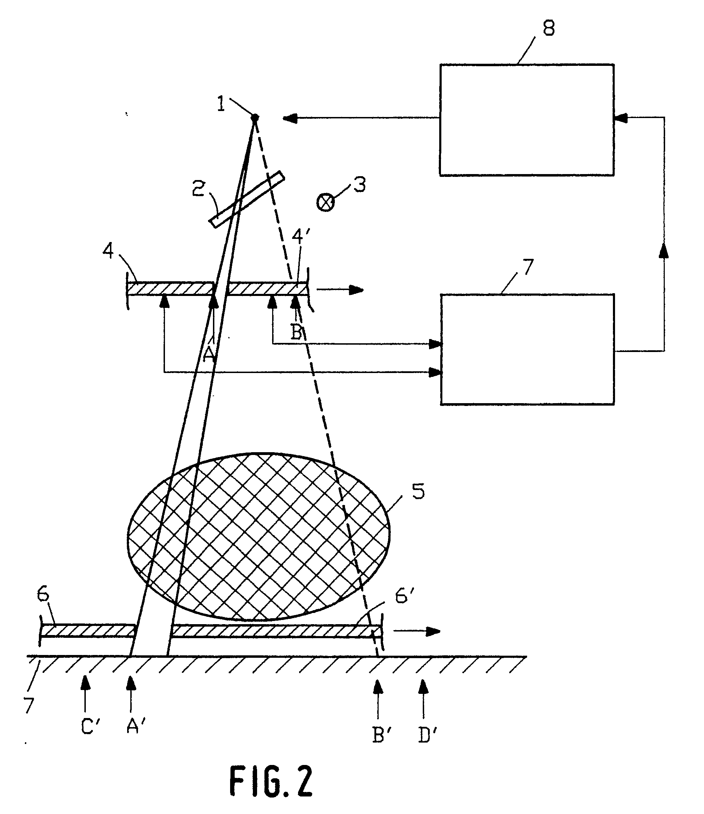

In der Darstellung gemäß Fig. 2, die die Aufnahmevorrichtung nach Fig. 1 zeigt, sind die beiden Blendenelemente 4 und 4′ der Primärspaltblende in Normalposition dargestellt, d.h. also derjenigen Position, in der sie eine geradlinige schlitzförmige Öffnung zur Belichtung des Untersuchungsobjektes mit Röntgenstrahlen bilden.

Während des Röntgenaufnahmevorgangs werden die beiden Blendenelemente 4 und 4′ der Primärspaltblende sowie die beiden Blendenelemente 6 und 6′ der Sekundärspaltblende in der Weise synchron zueinander bewegt, daß sie gemeinsam mit dem Fokus 1 der Röntgenstrahlenquelle in einer Ebene ausgerichtet bleiben.

Bei der Belichtung des Untersuchungsobjektes 5 mit Röntgenstrahlen steuert die Kontrollogik 7 den Röntgengenerator 8 und damit die Röntgenstrahlenquelle 1 in der Weise, daß eine Belichtung des Untersuchungsobjektes nur zwischen den Positionen A′ und B′ vorgenommen wird, welche den Begrenzungspostionen A und B der Blendenelemente 4 und 4' der Primärspaltblende entsprechen.

Gleichwohl kann der mechanische Bewegungsvorgang der Primärspaltblende und der Sekundärspaltblende in einem maximalen Untersuchsbereich vorgenommen werden, beispielsweise zwischen den Positionen C' und D' in der Ebene der Sekundärspaltblende. Es wird dann beispielsweise beginnend bei der Position C' in der Sekundärspaltebene sowohl der Primärspalt wie auch der Sekundärspalt synchron zueinander bewegt, wobei jedoch zunächst noch die Röntgenstrahlenquelle ausgeschaltet ist. Erst wenn das Blendenelement 4 der Primärspaltebene seine Begrenzungsposition A erreicht hat, wird die Röntgenstahlenquelle 1 eingeschaltet. Es tritt dann Röntgenstrahlung im Bereich des Pfeiles A' in der Sekundärspaltebene auf den Strahlungsdetektor 7. Nachfolgend werden die Blendenelemente 4 und 4' der Primärspaltblende sowie die Blendenelemente 6 und 6' synchron zueinander weiterbewegt bis das Blendenelement 4' seine Begrenzungsposition B erreicht hat. Sobald dies der Fall ist, wird über die Kontrollogik 7 der Röntgengenerator 8 ausgeschaltet, so daß keine Röntgenstrahlung von der Strahlenquelle 1 mehr ausgeht. Die Blendenelemente 4 und 4' der Primärspaltblende sowie 6 und 6' der Sekundärspaltblende können dann gegebenenfalls noch bis zu einer Extremposition D' weitergefahren werden.

Unabhängig davon, ob die Blendenelemente der beiden Blenden mechanisch jeweils nur in dem gewünschten Untersuchungsbereich oder zwischen Extrempositionen (z.B. gemäß Fig. 2, Positionen C' und D') bewegt werden, kommt es in jedem Falle darauf an, daß eine Belichtung des Untersuchungsobjektes nur in dem durch die Begrenzungspositionen der Primärspaltblendenelemente 4 und 4 festgelegten Untersuchungsbereich stattfindet, d.h. also, daß in jedem Falle die Röntgenstrahlenquelle nur dann eingeschaltet sein darf, wenn die Röntgenstrahlen in diesem Bereich auf das Untersuchungsobjekt treffen.

Es sei noch erwähnt, daß die Bewegung der Primärspalt-Blendenelemente 4 und 4' nicht notwendigerweise, wie in den Figuren dargestellt, linear geschehen muß; es kann auch vorgesehen sein, die Primärspalt-Blendenelemente in der Weise um den Fokus der Röntgenstrahlenquelle 1 herumzuschwenken, daß die Spalte der Primärblende und der Sekundärblende und der Fokus 1 der Röntgenstrahlenquelle fluchten.

**Ansprüche**

1. Röntgenaufnahmevorrichtung mit einer Röntgenstrahlquelle (1), deren Strahlung durch eine schlitzförmige Öffnung wenigstens einer Primärblende fächerförmig auf ein Untersuchungsobjekt (5) trifft und zeilenweise über dieses geführt wird, und mit einem Strahlungsdetektor (7),
dadurch gekennzeichnet , daß die Primärblende wenigstens zwei Blendenelemente (4, 4') aufweist, die in Normal-position die schlitzförmige Öffnung bilden und die zur Festlegung eines Untersuchungsbereichs quer zur Schlitzrichtung auf je eine wählbare Begrenzungsposition einstellbar sind, daß eine Lichtquelle (3) vorgesehen ist, deren Lichteinfall auf das Untersuchungsobjekt (5) mittels der in den Begrenzungspostionen befindlichen Blendenelemente (4, 4') begrenzt wird und so den Untersuchungsbereich markiert und daß eine Belichtung des Untersuchungsobjektes (5) mit Röntgenstrahlen nur zwischen den beiden Positionen der Primärblende vorgenommen wird, in denen sich deren Blendenelemente (4, 4') in ihrer jeweiligen Begrenzungsposition befinden.

2. Röntgenaufnahmevorrichtung nach Anspruch 1, dadurch gekennzeichnet , daß das Licht der Lichtquelle (3) mittels eines zwischen der Röntgenstrahlquelle (1) und der Primärblende angeordneten Umlenkspiegels (2) eingeblendet wird.

3. Röntgenaufnahmevorrichtung nach Anspruch 2, dadurch gekennzeichnet , daß die beiden Blendenelemente (4, 4') manuell auf die ihnen jeweils zugeordnete Begrenzungsposition einstellbar sind.

4. Röntgenaufnahmevorrichtung nach Ansproch 1 oder 2,
dadurch gekennzeichnet , daß die beiden Blendenelemente (4, 4') motorisch auf die ihnen jeweils zugeordnete Begrenzungsposition einstellbar sind.

5. Röntgenaufnahmevorrichtung nach Anspruch 3 oder 4,
dadurch gekennzeichnet , daß eine Kontrollogik (7) vorgesehen ist, welche die Begrenzungspositionen speichert und während einer Röntgenaufnahme die Röntgenstrahlquelle (1) nur während desjenigen Zeitraumes einschaltet, in dem sich die Blendenelemente (4, 4') der Primärblende zwischen den ihnen jeweils zugeordneten Begrenzungspositionen befinden.

FIG.1

FIG. 2